# EUROPÄISCHE PATENTSCHRIFT

(11) **EP 3 600 206 B1**
(45) Veröffentlichungstag und Bekanntmachung des Hinweises auf die Patenterteilung: **07.06.2023**
(21) Anmeldenummer: 17720364.3
(22) Anmeldetag: 03.04.2017
(51) Int. Cl.: A61G 11/00, A01N 1/02, A61M 1/36, A61G 10/02

(54) **KÜNSTLICHES GEBÄRMUTTERSYSTEM UND PLAZENTA**
ARTIFICIAL WOMB SYSTEM AND PLACENTA
SYSTÈME D'UTÉRUS SYNTHÉTIQUE ET PLACENTA

(30) Priorität: 21.03.2017 WO PCT/EP2017/056704
(43) Veröffentlichungstag der Anmeldung: 05.02.2020
(73) Patentinhaber: Tchirikov, Michael, 06126 Halle (Saale) (DE)
(72) Erfinder: Tchirikov, Michael, 06126 Halle (Saale) (DE)
(74) Vertreter: Patentanwälte Dr. Keller, Schwertfeger Partnerschaft mbB
(86) Internationale Anmeldenummer: PCT/EP2017/057829
(87) Internationale Veröffentlichungsnummer: WO 2018/171905

(56) Entgegenhaltungen:
- WO-A1-2014/145494
- WO-A1-2014/145494
- WO-A1-2016/205622
- WO-A1-2016/205622
- WO-A2-2007/008840
- US-A- 5 207 639
- US-A- 5 207 639
- US-A1- 2007 010 005

## Beschreibung

### Technisches Gebiet:

Geburten von Kindern unter der 24. Schwangerschaftswoche (SSW) mit einem Gewicht von weniger als 500 g, bei denen kein Lebenszeichen auftritt, gelten als "Spätabort" und werden statistisch als Geburten nicht erfasst. Weltweit sind dabei mehrere Millionen Kinder in den entwickelten Ländern betroffen. Von allen extrem Frühgeborenen (d.h. unter der 28. SSW) versterben 40 % innerhalb der ersten fünf Jahre (WAO-Bericht "Born to soon", 2011). Es sterben 91 % der Frühgeborenen in der 23. SSW und 67 % in der 24. SSW (Stoll et al. JAMA 2015). Nur selten überleben extrem Frühgeborene ohne gravierende Spätfolgen (Chen F. et al. Arch Dis Chld Fetal Noanatal 2016; 101:377-83). Nur 6 % aller Frühgeborenen in der 22. SSW überleben bis zu ihrer Entlassung aus dem Krankenhaus, wobei 95 bis 96 % davon ausgeprägte körperliche und/oder geistige Schäden aufweisen (Stoll et al. JAMA 2015). Mit 22 Schwangerschaftswochen haben die überlebenden Frühgeborenen in 89 % der Fälle eine Retinopathie mit einem Schweregrad > 3. In der 23. SSW sind 42 % betroffen. Lediglich ein Prozentsatz von < 20 % der Frühgeborenen überlebt bis zur Entlassung aus dem Krankenhaus ohne Ausbildung einer nekrotisierenden Enterokolitis, Sepsis, Meningitis, bronchiopulmonale Hypoplasie und/oder ausgeprägte Hirnblutung bei Frühgeburten unter der 24. SSW (Stoll et al. JAMA 2015). Ein ähnliches Bild mit zum Teil drastisch schlechten Überlebensraten liefert auch eine kürzliche Studie bei Neugeborenen zwischen der 22. und der 24. SSW (Noelle Younge et al., Survival and Neurodevelopmental Outcomes among Periviable Infants, The New England Journal of Medicine, February 16, 2017 vol. 376 no. 7).

### Stand der Technik:

Um diesem Problem entgegenzutreten, werden Methoden und Therapien entwickelt, um die Überlebensrate von extrem Frühgeborenen zu erhöhen und Spätfolgen bzw. ein verfrühtes Ableben zu vermindern. Ein Ansatz besteht darin, die Frühgeborenen in einen Neugeborenen-Inkubator nach der Entbindung von der Mutter aufzunehmen, um eine "künstliche" Gebärmutter bereitzustellen. Dabei sollen multiple Plazentafunktionen der Gebärmutter zumindest vorübergehend möglichst vollständig ersetzt werden. Ziel ist es, nicht nur die Überlebensrate zu erhöhen, sondern auch die im Rahmen der fetalen Programmierung einhergehenden bleibenden gravierenden Komplikationen einer extremen Frühgeburt zu vermeiden.

Die menschliche Plazenta ist ein kompliziertes fetales Organ, das zum einen die wichtige Versorgungsfunktion für das Embryo bzw. Fötus übernimmt. Dazu gehört beispielsweise ein transplazentarer aktiver und/oder passiver Transport von Sauerstoff, Aminosäuren, Fettsäuren, Mikroelementen, Vitaminen, Wasser, Elektrolyten, Wachstumsfaktoren, Hormonen, Zytokinen und anderen regulierenden Substanzen (NO etc.). Zum Teil werden auch Substanzen wie Aminosäuren, Hormone, NO in der Plazenta selbst synthetisiert. Da die fetalen Nieren "in utero" noch keine Entsorgungsfunktion haben, produzieren sie eine verdünnte hypoosmotische alkalische Flüssigkeit, die sich als Hauptanteil des gesamten Fruchtwassers darstellt. Ein Fötus trinkt täglich etwa 250 bis 300 ml/kg Fruchtwasser (Gilbert WM and Brace RA. Semin Perinatol. 1993). Die Entsorgung der fetalen Metaboliten wie z.B. Bilirubin oder CO₂ erfolgt über die Plazenta. Danach übernehmen die mütterlichen Nieren und Lungen die Entsorgungsfunktion.

Die Plazenta stellt ferner eine wichtige Barriere für mütterliche Bakterien und Viren dar, um diese von dem sich entwickelnden Kind fernzuhalten. Lediglich die IgG-Antikörper sind plazentadurchgängig.

Das Amnion oder der Fruchtsack, welches bereits ab der 4.-5. SSW das Embryo mitsamt seiner Nabelschnur umgibt, besteht aus 2 Blättern: amniale und choriale Membranen. Die Funktion ist hauptsächlich die Begrenzung zwischen dem Fötus und der Gebärmutter (Bernischke et al. Pathology of the human Placenta. Springer Verlag, 2012).

In der amnialen Membran werden Zytokine und Prostaglandine synthetisiert und sie spielt teilweise bei der Fruchtwasserproduktion eine Rolle. Das Fruchtwasser ist auch bei der direkten Übertragung von mütterlichen Geräuschen, beispielsweise Herzschlag, Atmung, Darmgeräuschen, aber auch der mütterlichen Stimme, direkt zum Ohr des Fötus beteiligt. Künstliche Lebenserhaltungssysteme, Neugeborenen-Inkubatoren bzw. künstliche Gebärmuttern sind bekannt. So beschreibt das US-Patent 5,218,958 ein Lebenserhaltungssystem für ein Frühgeborenes, das in der natürlichen mütterlichen Plazenta über dessen Nabelschnur eingebettet ist. Das System umfasst obere und untere Kammern, die durch eine gewölbeartige Zwischenwand voneinander abgetrennt sind. Die untere Kammer enthält physiologische Flüssigkeit, in der das Frühgeborene eingelegt ist, während die obere Kamer eine sauerstoffhaltige Atmosphäre sowie Nährstoffzugänge aufweist, um die Plazenta mit Nährstoffen zu versorgen. In diesem System soll die grundlegendste physiologische Versorgung des Frühgeborenen ermöglicht werden.

Die WO 2012/093087 A1 beschreibt eine wässrige Zusammensetzung mit verringertem Chloridgehalt, die als Fruchtwasserersatz verwendet werden kann. Der Aufbau eines Lebenserhaltungssystems für Frühgeborene oder einer künstlichen Gebärmutter ist nicht beschrieben.

Die US 2014/0255253 A1 beschreibt eine künstliche Gebärmutter, die mit einer Sauerstoffversorgungseinrichtung für den Frühgeborenen ausgerüstet ist. Die Vorrichtung umfasst eine für Gas permeable Membran und ein vaskuläres Netzwerk, über das beispielsweise Flüssigkeiten mit Nährstoffen zugeleitet werden können. Die Sauerstoffversorgung erfolgt über einen Oxygenator über die Nabelschnur unter Verwendung eines umbilikalen Katheters (Nabelschnurkatheter), der einen venösen Katheter und einen arteriellen Katheter umfasst. Über dieses Kathetersystem wurde ein Gasgemisch mit 40 % Sauerstoff in Stickstoff verarbreicht. Die Sicherung des Katheters oder dessen genauer Aufbau ist nicht beschrieben.

Eine andere Möglichkeit der Oxygenierung eines Frühgeborenen beschreibt die WO 2014/145494 A1, bei der das Kreislaufsystem des Frühgeborenen an eine extrakorporale Membran gekoppelt wird, welche Teil eines Sauerstoffversorgungssystems ist. Eine Variante sieht vor, dass der Oxygenator mit fetalem Blut angereichert ist. Die Inkubationskammer umfasst eine sterile Flüssigkeit und kann bedarfsweise aufgewärmt werden. Bei dem System ist vorgesehen, dass das Frühgeborene intravenös in der Inkubationskammer mit Nährstoffen versorgt wird. Ferner sind bei der Inkubationskammer Einlässe und Auslässe und bedarfsweise auch eine Pumpe mit wenigstens einem Filter vorgesehen. Das Infektionsrisiko und das Risiko zur Ausbildung einer Sepsis sind sehr groß.

Die DE 20 2014 103 422 U1 beschreibt einen eine Gebärmutter nachbildenden Inkubator mit einem Lumen zur Beherbergung eines Neugeborenen und eines Umweltänderungssystems, das so konfiguriert ist, dass zumindest ein Parameter in einem vorbestimmten Zeitzyklus verändert wird, um die menschliche innergebärmütterliche Umwelt nachzubilden. Dazu gehört beispielsweise ein Lichtfilter, ein Geräuschdämpfer, ein Stoßdämpfer. Ferner können auch Parameter erzeugt werden, wozu beispielsweise ein Lichtsystem, ein Geruchssystem, ein Geräuschsystem oder ein Bewegungssystem gehört. Auf die physiologischen Anforderungen eines in einer Gebärmutter eingebetteten extrem Frühgeborenen wird nicht eingegangen. Vielmehr geht es darum, eine gleichbleibende, sichere und ruhige Umwelt für ein Neugeborenes bereitzustellen.

Die WO 2016/154319 A1 beschreibt eine künstliche Gebärmutter und Verfahren zu deren Herstellung, bei der Mikrofluidkanäle vorgesehen sind, die zwischen einer Membran derart angeordnet sind, dass ein Flüssigkeitstransport durch die Membran erfolgen kann. Ferner sind Zellschichten von wenigstens zwei unterschiedlichen Zelltypen vorgesehen, welche an den beiden Seiten der Membran haften. Bei dem ersten Zelltyp handelt es sich beispielsweise um primäre humane plazentale villöse Endothelzellen, während der zweite Zelltyp Choriokarzinomzellen umfasst.

Die im Stand der Technik beschriebenen Gebärmuttersysteme sind in vielen Punkten unzureichend, zum einen, weil keine in physiologischer Hinsicht gleichkommenden Bedingungen herrschen oder weil die Behandlung des extrem Frühgeborenen ungeeignet ist.

So beschreibt das US-Patent 5,218,958 eine physiologische Lösung, bei der das Gewebe der Plazenta innerhalb von 24 Stunden anschwillt und die Funktion komplett lahmlegt. Die beschriebene Lösung ist für das Kind und dessen Lebenserhaltung auch schon deshalb ungeeignet, weil der Anteil von Na+ und CI-Ionen viel zu hoch ist. Auch setzt das Konzept voraus, dass die Plazenta mit der Nabelschnur verbunden ist. Die Weiterverwendung einer abgelösten Plazenta für die Versorgung des Frühgeborenen hat sich in der Praxis nicht durchgesetzt. Eine vorzeitige Plazentaablösung führt zu einem intrauterinen Fruchttod. Die Plazenta zieht sich nach der Lösung zusammen und verliert ihre Funktion. Nach der Geburt wiederum entstehen in der Plazenta mehrere Risse, die zu einem kontinuierlichen Blutverlust beim Frühgeborenen führen.

Die in der WO 2014/145494 A1 beschriebenen Fruchtwasserersatzlösungen sind schon allein wegen eines zu niedrigen pH-Wertes, einer erhöhten Osmolarität, des Fehlens von Mikroelementen, Glucose und einer unzureichenden NaCl-Konzentration ungeeignet.

Für den Ersatz der Lungenfunktion wird häufig eine extrakorporale Membranoxygenierung (ECMO) durchgeführt, die normalerweise veno-venös erfolgt. Daneben kennt man auch CLS-Systeme (extracorporal life support), die eine extrakorporale Oxygenierung und CO₂-Eliminierung ermöglichen, wodurch die Lungenfunktion ersetzt werden kann. Mit der ECLS ist auch eine hämodynamische Entlastung des Herzens möglich, da es zu einer Kreislaufunterstützung kommt. Bei den ECMO-Systemen werden ein veno-arterielles ECLS und eine veno-venöse ECMO unterschieden. Das Blut wird aus dem venösen System des Patienten mittels einer Pumpe befördert und nach Passage durch den Oxygenator wieder dem arteriellen System zugeführt. Die Sauerstoffversorgung des Organismus setzt sich aus dem ECMO/ECLS-Fluss und der verbleibenden Kreislauffunktion des Patienten zusammen. Die Verbindung mit dem extrakorporalen Kreislauf erfolgt normalerweise über Kanülen. Bei Neugeborenen und Kleinkindern werden hierfür häufig die Halsgefäße (d.h. die Arterie carotis bzw. Vena jugularis) zur Kanülisierung herangezogen, da sie den größten Durchmesser haben. Dies könnte jedoch zu irreversiblen Schäden aufgrund einer Minderperfusion des Gehirns führen.

Die WO 2016/205622 A1 beschreibt ein Verfahren und eine Vorrichtung, die konfiguriert sind, um eine extrakorporale Unterstützung für Frühgeborene bereitzustellen. Hierbei ist der Fruchtwasserkreislauf so konfiguriert, dass das Fruchtwasser in die Fruchtkammer eindringen und aus dieser auch wieder entweichen kann, um einen Flüssigkeitsdruck in der Fruchtkammer innerhalb eines vorbestimmten Bereiches aufrechtzuerhalten. Der Fruchtkreislauf kann ein geschlossenes System sein.

### Darstellung der Erfindung:

Vor diesem Hintergrund ist es Aufgabe der vorliegenden Erfindung, ein verbessertes künstliches Gebärmuttersystem zur Lebenserhaltung von Neugeborenen, insbesondere extrem Frühgeborenen zwischen der 21/0 und 28/0 SSW bereitzustellen, mit dem die Gesamtlebenserwartung des Frühgeborenen gesteigert werden kann.

Diese Aufgabe wird gelöst durch ein künstliches Gebärmuttersystem zur ex-vivo-Behandlung eines Neugeborenen oder Frühgeborenen, wie in den nachfolgenden Ansprüchen beansprucht. Bevorzugte Ausführungsformen finden sich in den Unteransprüchen wieder.

Das erfindungsgemäße künstliche Gebärmuttersystem dient zur Lebenserhaltung von Neugeborenen, die ohne Hilfe nicht lebensfähig wären, insbesondere extrem Frühgeborenen zwischen der 21/0 und 28/0 SSW (x/y SSW = Schwangerschaftswoche/Tage), d.h. vorzugsweise von Frühgeborenen, die vor der vollendeten 28. Schwangerschaftswoche geboren sind. Das Gebärmuttersystem umfasst zunächst einen von einer zumindest teilweise ultraschalldurchlässigen Wandung gebildeten künstlichen Raum der künstlichen Gebärmutter mit einem Lumen zur Aufnahme von Fruchtwasser und eines Frühgeborenen. Durch die Verwendung eines ultraschalldurchlässigen Materials für die Wandung des Raums der Gebärmutter sind Ultraschallmessungen möglich, die vorzugsweise mit Sonden in einem Frequenzbereich von 1 bis 15 MHz, vorzugsweise in einem Frequenzbereich von 3,5 bis 12 MHz oder Bereichen dazwischen durchgeführt werden. Bevorzugte Materialien für die Wandung des Raums der künstlichen Gebärmutter sind daher transparente Kunststoffe oder andere ultraschalldurchlässige Materialien. In einer bevorzugten Variante ist die Wandung auch lichtdurchlässig. Vorzugsweise besteht die Wandung aus einem Material bestehen, das entweder von sich aus über Abschattung des Innenraums dunkel gefärbt ist oder bei Lichteinwirkung lichtabweisend wirkt.

Das System umfasst ferner wenigstens einen Zugang zur Versorgung des Frühgeborenen in dem Raum der künstlichen Gebärmutter mit Fruchtwasser. Vorzugsweise sind in dem Fruchtwasser Nährstoffe, Hormone, Wachstumsfaktoren oder andere lebenswichtigen Substanzen und Verbindungen gelöst.

Da die Nierenfunktion des Neugeborenen oder des extrem Frühgeborenen noch nicht ausgebildet ist, ist ferner eine Dialyseeinrichtung vorgesehen, um die Abbauprodukte des Frühgeborenen zu entsorgen. Ein Oxygenator und/oder eine Begasungseinrichtung sorgt für die Sauerstoffversorgung des Frühgeborenen, entweder mit Sauerstoff, einem Sauerstoffhaltigen Gasgemisch oder mit Carbogen^{®}.

Der Erfinder der vorliegenden Erfindung hat festgestellt, dass die im Stand der Technik beschriebenen Vorrichtungen und Methoden schon allein unzureichend sind, da sie den intraaminalen Druck in der Gebärmutter nicht berücksichtigen. Erfindungsgemäß ist daher vorgesehen, dass Mittel vorgesehen sind, um einen auf das Neugeborene wirkenden intraamnialen Druck in dem Raum der künstlichen Gebärmutter von >0 mBar zusätzlich zum Atmosphärendruck aufrechtzuerhalten. Vorzugsweise wird in dem Raum der künstlichen Gebärmutter ein auf das Neugeborene wirkender Druck zwischen 2 mBar und 1000 mBar, bevorzugt zwischen 5 mBar und 600 mBar, bevorzugt zwischen 5 mBar und 100 mBar, oder Bereiche dazwischen, zusätzlich zu dem Atmosphärendruck aufrechterhalten. Der Atmosphärendruck beträgt üblicherweise 1 Bar (ca. 1013 hPa). Der Absolutdruck in der künstlichen Gebärmutter beträgt demnach vorzugsweise bis zu 2 Bar oder beliebige Werte dazwischen. Alternativ ist auch eine Beaufschlagung mit einem Absolutdruck von 5 mBar bis 2 Bar möglich. Auch Zwischenbereiche oder Zwischenwerte der oben genannten Bereiche sind von der Erfindung umfasst. Für die Aufrechterhaltung des intraamnialen Druckes sind Einrichtungen oder Mittel vorgesehen, wie beispielsweise eine Pumpe oder ein unter Druck stehendes Behältnis, das mit künstlichem Fruchtwasser oder einem Sauerstoffgemisch gefüllt ist.

Erfindungsgemäß wird die intraamniale Druckerhaltung in der künstlichen Gebärmutter in einer bevorzugten Variante dadurch gelöst, dass eine Pumpe für den Transport von Fruchtwasser und/oder für die Fruchtwasserbegasung vorgesehen ist. Diese pumpt Fruchtwasser, Carbogen^{®} oder ein sauerstoffhaltiges Gemisch in den Raum der künstlichen Gebärmutter. Alternativ kann auch ein unter Druck stehendes Behältnis (z.B. gefüllt mit Fruchtwasser oder einem Sauerstoffgemisch) verwendet werden, um den gewünschten intraamnialen Druck in der künstlichen Gebärmutter aufzubauen. Der Aufbau des auf das Neugeborene wirkenden Druckes kann auch durch eine Flüssigkeitssäule erfolgen, beispielsweise in dem Raum gefülltes Fruchtwasser, das auf das Neugeborene einwirkt und einen Druck > 0 mBar, vorzugsweise > 0 mBar und 1 Bar, zusätzlich zum Atmosphärendruck (1 Bar) aufbaut. Alternativ kann der Druckaufbau in dem Raum auch über eine Begasung erfolgen. Je nachdem welche Mittel für den intraamnialen Druckaufbau gewählt werden, kann der Raum geschlossen oder zumindest zu einer Seite offen sein. Ein (vorzugsweise nach oben) offener Raum wäre beispielsweise bei Verwendung von (Frucht)-Wasser denkbar, in das das Neugeborene eingebettet ist.

Der Oxygenator und die Dialyseeinrichtung sind mit einem Portsystem verbunden, welches wiederum mit der Nabelschnur des Frühgeborenen verbunden ist. Das erfindungsgemäße Portsystem besteht aus wenigstens zwei Kathetern zur Verbindung der Umbilikalvene und wenigstens einer Nabelschnurarterie des Frühgeborenen. Vorzugsweise umfasst das erfindungsgemäße Portsystem einen Katheter für die Nabelschnurvene und zwei Katheter für die beiden Nabelschnurarterien. Ferner ist ein mit den Kathetern verbundener Stent vorgesehen, über den eine Verankerung des Portsystems mit der Nabelschnur erfolgt.

Der Erfinder hat ferner erkannt, dass der beste physiologische Zugang zum Kreislauf des extrem Frühgeborenen die Nabelschnur ist. Problematisch ist jedoch, dass die Nabelschnur kurz nach der Geburt, normalerweise innerhalb von 3 bis 4 Tagen, abfällt. Das macht eine Dauerverwendung der Nabelschnur zur Versorgung des Frühgeborenen scheinbar unmöglich. Auf der anderen Seite ist eine Dauer-Katheterisierung der intrahepatischen Umbilikalvene über den Nabel mit einem erheblichen Risiko zur Entwicklung einer Omphalitis oder Sepsis des Frühgeborenen verbunden.

Das erfindungsgemäße Portsystem ermöglicht zur Lösung dieses Problems eine Fixierung wenigstens eines venösen und eines arteriellen Katheters, vorzugsweise eines venösen und zwei arterieller Katheter, an die Nabelschnur, um auch im Falle einer Kindsbewegung in dem Raum der künstlichen Gebärmutter ein unbeabsichtigtes Abreißen zu vermeiden.

Vorzugsweise befindet sich ein Teil des Portsystems im Fruchtwasser der künstlichen Gebärmutter, was auch einer möglichen Drucknekrose der Nabelschnur vorbeugt. Dazu ist das erfindungsgemäße Portsystem vorzugsweise wasserdicht ausgeführt. Vorzugsweise umfasst der Katheter für die Umbilikalvene einen Durchmesser zwischen 3 und 10 mm, während die Katheter für die beiden Nabelschnurarterien vorzugsweise einen Durchmesser von 2 bis 7 mm oder auch Bereiche dazwischen umfassen. Vorzugsweise ist das erfindungsgemäße Portsystem mit einem ECMO-Gerät als Oxygenator verbunden, wobei die Anreicherung mit Sauerstoff vorzugsweise mit gespendetem fetalem Nabelschnurblut erfolgt. Die Überführung des Frühgeborenen erfolgt durch eine Ablösung der natürlichen Plazenta von der Nabelschnur und ein sofortiges Einbetten in dem mit künstlichem Fruchtwasser gefüllten Raum der künstlichen Gebärmutter des erfindungsgemäßen künstlichen Gebärmuttersystems. Der Stent des Portsystems ist vorzugsweise am Ende des Schlauchsystems angeordnet, um den Durchmesser des katheterisierten Gefäßes nicht unnötig zu reduzieren, eine sichere Fixierung zu garantieren und um auch die spätere Fixierung der Nabelschnur zum ECMO-Zugang/-Ausgang des künstlichen Gebärmuttersystems zu ermöglichen.

Vorzugsweise sind die Katheter des Portsystems durch einen Halter geführt, der so ausgeführt ist, dass er die Nabelschnur des Frühgeborenen von der Außenseite voll umfänglich umgreift. Der erfindungsgemäße Stent des Portsystems ist vorzugsweise so ausgeführt, dass die Katheter mit der Gefäßinnenwand der Nabelschnur verbindbar sind. Vorzugsweise erfolgt die Fixierung des Portsystems über den Stent über eine Vielzahl von kleinen Greifelementen oder Häkchen, die mit der Gefäßinnenwand der Nabelschnur des Frühgeborenen verrasten. Die damit einhergehende Möglichkeit zur Entwicklung einer aseptischen Drucknekrose ist in diesem Fall unkritisch, da die Nabelschnur in regelmäßigen Abständen, vorzugsweise alle drei bis fünf Tage, spätestens jedoch nach sieben Tagen, in dem stentisierten Bereich abgeschnitten wird. Eine Re-Kathetisierung der Nabelschnurgefäße vermeidet zudem eine Nekrose und bakterielle oder virale Infizierung und Kolonisierung des distalen Nabelschnurendes. Der abgeschnittene Stumpf der Nabelschnur kann einer mikrobiologischen Untersuchung unterzogen werden. Die menschliche Nabelschnur bietet aufgrund ihrer Länge von 60 bis 80 cm die Möglichkeit, einfach und effizient eine Re-Kathetisierung in regelmäßigen zeitlichen Abständen durchzuführen, wobei die Reduzierung vorzugsweise zwischen 3 und 5 cm ausmacht.

Vorzugsweise umfasst das erfindungsgemäße Portsystem eine Klammer zur Fixierung des Ports mit der Nabelschnur von außen. Neben einer Sauerstoffversorgung über das erfindungsgemäße Portsystem, welche direkt über das venöse-arterielle System der Nabelschnur bei gleichzeitiger CO₂-Reduzierung erfolgt, ist in einer bevorzugten Variante des Gebärmuttersystems auch eine transkutane fetale Versorgung über eine Carbogen^{®}- oder Sauerstoff-Begasung möglich. Die Carbogen^{®}-Begasung sieht ein Gasgemisch vor, das aus etwa 5 Volumenteilen Kohlenstoffdioxid (CO₂) und 95 Volumenteilen Sauerstoff (O₂) besteht. Vorzugsweise beaufschlagt der Oxygenator der künstlichen Gebärmutter direkt mit dem Carbogen^{®}-Gasgemisch oder Sauerstoff. In einer alternativen Variante erfolgt eine Carbogen^{®}-oder O₂-Behandlung des der künstlichen Gebärmutter zugeführten Fruchtwassers entweder vor der Zuführung in den Raum der künstlichen Gebärmutter oder durch Behandlung von Fruchtwasser, welches sich bereits im Raum der künstlichen Gebärmutter befindet. Die Aufnahme von Sauerstoff durch das extrem Frühgeborene kann daher wahlweise über die Nabelschnur oder durch Begasung über die Haut erfolgen.

Die Erfassung der O₂-Sättigung in dem Fruchtwasser der künstlichen Gebärmutter erfolgt vorzugsweise über eine Messeinrichtung. Ferner kann über die erfindungsgemäß vorgesehene Messeinrichtung auch der Druck und/oder die Temperatur oder eine Sonographie vorgenommen werden. Das der künstlichen Gebärmutter zugeführte Fruchtwasser wird von einer Fruchtwasservorwärmeinrichtung auf eine Temperatur von vorzugsweise 37° bis 39° C vorgewärmt. Über eine entsprechende Wärmeeinrichtung kann der Raum der künstlichen Gebärmutter bevorzugt auf eine Temperatur zwischen 37° und 38° C gehalten werden.

Die künstliche Gebärmutterumfasst in einer bevorzugten Variante Anschlüsse für ECMO/ECLS-Schläuche zur Fruchtwassereinleitung und Fruchtwasserableitung, zur Fruchtwasserbegasung und/oder zur Harnableitung. Für eine erfolgreiche Lebenserhaltung und Weiterentwicklung des Frühgeborenen ist auch die Zusammensetzung des künstlichen Fruchtwassers entscheidend. Bei einer unzureichenden Fruchtwasserzusammensetzung besteht die Gefahr, dass fetale innere Organe, Nabelschnur, Amnion, Haut, Augen oder Schleimhäute irreversible Schäden erleiden.

Der Erfinder hat ferner festgestellt, dass Föten unterhalb der 28. SSW nicht in der Lage sind, die kommerziell angebotenen Aminosäuren umzusetzen, zu metabolisieren bzw. zu vertragen (Tchirikov M et. al. J Perinat Med. 2016). Die bislang eingesetzten kommerziellen Lösungen beinhalten Aminosäurekonzentrationen, die sich von den physiologischen Verhältnissen im fetalen Plasma zum Teil sehr drastisch (teilweise bis zu 100fach) abheben (Tchirikov M et. al. J Perinat Med. 2016). Einige Aminosäuren sind sogar bei kommerziellen Präparaten gar nicht vorhanden. Wird eine solche Aminosäuresubstitution durchgeführt, erfolgt bei dem Fötus unter 1000 g kein Wachstum. Die Hälfte dieser Föten stirbt intrauterin oder innerhalb einer Woche nach der Entbindung ab.

Das erfindungsgemäß eingesetzte Fruchtwasser umfasst daher in einer bevorzugten Ausführungsform eine Nährstoffzusammensetzung, deren Konzentrationen mit der physiologischen Situation beim Fötus im jeweiligen Gestationsalter übereinstimmen. Dadurch wird die Konzentration von Fettsäuren, Vitaminen, Mikroelementen, Wachstumsfaktoren, Hormonen, Elektrolyten, Zytokinen und anderen regulatorischen Substanzen ständig überprüft, angepasst und bei Bedarf substituiert. Vorzugsweise umfasst der Raum der künstlichen Gebärmutter des erfindungsgemäßen Gebärmuttersystems künstliches Fruchtwasser, welches folgende Zusammensetzung aufweist.

| | Konz. |
|---|---|
| Osmolarität [mosm/l] | 240-300 |
| Natrium [mmol/l] | 130-145 |
| Kalium [mmol/l] | 3,5-4,5 |
| Calcium [mmol/l] | 1-2,5 |
| Magnesium [mmol/l] | 0-2 |
| Chlorid [mmol/l] | 100-120 |
| Harnstoff [mg/dl] | 0-10 |
| Harnsäure [mg/dl] | 0-5 |
| Phosphat [mg/dl] | 0-8 |
| Glucose [mg/dl] | 0-340 |
| Laktat [mmol/l] | 0-20 |
| Citrat [mg/l] | 0-100 |
| Hydrogencarbonat [mmol/l] | 5-100 |
| Gesamt-Eiweiß [g/l] | 0-10 |
| Albumin [g/dl] | 0-5 |
| Kupfer [µg/dl] | 0-150 |
| Selen [µg/dl] | 0-50 |
| Zink [µg/dl] | 0-30 |
| Lipide insgesamt [mg/l] | 0-2000 |
| Phosphatidylcholin (PC) [mg/l] (bevorzugt 80% der Lipide insgesamt) | 0-1000 |

Es sind vorzugsweise andere Spurenelemente, wie z.B. Bor, Chrom, Eisen, Fluor, Jod, Kobalt, Lithium, Mangan, Molybdän, Nickel, Silicium, Vanadium, Aminosäuren, Wachstumsfaktoren, Vitamine und Hormone enthalten, um eine physiologische Entwicklung des Frühgeborenen zu gewährleisten. Vorzugsweise wird das Fruchtwasser vor der Einleitung in die künstliche Gebärmutter auf eine Temperatur zwischen 37 und 39° C vorgewärmt und gleichzeitig mit Sauerstoff oder einem sauerstoffhaltigen Gasgemisch begast. Vorzugsweise ist hierfür ein Behälter vorgesehen, der einen Druck von bis zu 2 bar halten kann. Dazu sind vorzugsweise Druckmessgeräte in der Wand integriert. Daneben sind Systeme zur Messung der fetalen O₂-Sättigung, EKG-Elektroden und Temperatursonden vorgesehen. Die fetale O₂-Sättigung wird nach der Abnabelung bei 70-80% in dem künstlichen Gebärmuttersystem durch den Oxygenator und/oder ggf. durch Begasung des Fruchtwassers gehalten. Dann erfolgt eine dosierte Steigerung der O₂-Sätigung auf 89-94%.

In einer bevorzugten Variante umfasst das Gebärmuttersystem auch wenigstens einen Zugang für die Vornahme einer zusätzlichen künstlichen Beatmung, die ab der 23./24. SSW anfallen könnte. Die Temperatur im Raum der künstlichen Gebärmutter wird vorzugsweise bei einer Temperatur zwischen 36 und 38° C von einer Temperaturkontrolleinrichtung konstant gehalten, bevorzugt zwischen 36,5 und 37,5°C. Die Funktion der Körperkühlung soll auch im Falle einer Asphyxie vorhanden sein. In einer bevorzugten Variante ist vorgesehen, dass der Raum der künstlichen Gebärmutter auf einer Bewegungseinrichtung gelagert ist. Dadurch soll die Mutterbewegung auf den Fötus reproduziert werden. Bedarfsweise kann auch eine Waage vorgesehen sein, um regelmäßig Gewichtsmessungen beim Fötus durchführen zu können.

In einer weiteren bevorzugten Variante ist vorgesehen, dass in der künstlichen Gebärmutter Muttergeräusche über eine Audioeinrichtung eingespielt werden. Bei den Muttergeräuschen handelt es sich beispielsweise um die mütterliche Stimme, die Atmung, Darmgeräusche und Herzschlag. Diese werden vorzugsweise von der Mutter von einer Aufnahmevorrichtung aufgenommen und über eine Audioeinrichtung in den Raum der künstlichen Gebärmutter zum Frühgeborenen eingespielt. Daneben können auch die mütterliche Stimme oder vorgesungene Lieder direkt beim Elternbesuch eingespielt werden, wofür die Wandung des Raums der künstlichen Gebärmutter vorzugsweise schalldurchlässig ausgeführt ist. Zwar ist grundsätzlich das Einspielen von Musik oder Geräuschen bei Patienten bekannt, jedoch umfassen solche Varianten keine Bespielung mit internen Muttergeräuschen wie beispielsweise die Atmung, Darmgeräusche oder den Herzschlag. Ziel der erfindungsgemäßen Audioeinwirkung ist es, die fetale Programmierung zu normalisieren und gegebenenfalls in positive Richtung zu lenken. Auch soll die Produktion von katabolischen Stresshormonen (beispielsweise Adrenalin, Noradrenalin oder Cortison) reduziert werden. In einer bevorzugten Variante ist auch die Kombination unterschiedlicher Geräusche möglich.

Es wird offenbart ein Verfahren zur ex-vivo-Behandlung eines Neugeborenen, vorzugsweise eines extrem Frühgeborenen zwischen der 21/0 und 28/0 SSW zur Aufrechterhaltung von dessen Lebensfunktionen. Vorzugsweise ist die Frühgeburt vor der vollendeten 28. Schwangerschaftswoche. Das erfindungsgemäße Gebärmuttersystem und das Verfahren, das nicht Teil der Erfindung ist, funktionieren jedoch auch bei Normalgeborenen, d.h. Neugeborenen, die eine eingeschränkte Lebenserhaltung haben, beispielsweise durch angeborene Defekte oder aufgrund noch nicht ausgebildeter Funktionen. Hierzu zählen beispielsweise Neugeborene mit einer Hypoplasie der Lunge. Bei diesen Kindern kommt eine ECMO-Behandlung über die Nabelschnur in Frage. Vorteilhaft ist hierbei, dass das Kind und damit die Nabelschnur beim erfindungsgemäßen Gebärmuttersystem in künstlichem Fruchtwasser eingebettet ist. So erleidet das Neugeborene bzw. extrem Frühgeborene keine Schmerzen, beispielsweise durch Benutzung von Kanülen oder Katheterisierungen.

Hierfür wird in einem ersten Verfahrensschritt ein zumindest teilweise ultraschalldurchlässiger Raum mit einem Lumen zur Aufnahme von Fruchtwasser und eines Neugeborenen, vorzugsweise eines extrem Frühgeborenen vor der vollendeten 28. SSW, bereitgestellt. Anschließend erfolgt eine Versorgung des Neugeborenen bzw. des Frühgeborenen in dem Gebärmuttersystem mit Nährstoffen und Sauerstoff. Die Ableitung von fetalen Abfallprodukten erfolgt über eine Dialyseeinrichtung.

Es ist nun vorgesehen, dass in dem Lumen der künstlichen Gebärmutter ein intraamnialer Druck von > 0 mBar, vorzugsweise zwischen 2 mBar und 1000 mBar, vorzugsweise zwischen 5 mBar und 600 mBar, bevorzugt zwischen 5 mBar und 100 mBar, zusätzlich zu dem Atmosphärendruck (1 Bar) aufrechterhalten wird, wobei auch sämtliche Zwischenbereiche oder Zwischenwerte von der vorliegenden Erfindung umfasst sind. In einer bevorzugten Variante wird in die künstliche Gebärmutter vorgewärmtes künstliches Fruchtwasser, vorzugsweise 2 I bis 20 I täglich, eingeleitet. Sofern das Lumen geschlossen ist, erfolgt die Beaufschlagung des Druckes über verschiedene Mittel (z.B. Begasung oder Flüssigkeitszufuhr) direkt in dem Lumen. Bei einer Variante kann das Lumen auch zu einer Seite offen sein, so dass der Druck unmittelbar auf das Neugeborene wirkt. Auch hier ist die Maßgabe, dass der Druck > 0 mBar zusätzlich zum Umgebungsdruck (Atmosphärendruck) sein soll. Die medizinische Obergrenze dürfte sicherlich bei einem Absolutdruck von 2 Bar sein, sofern nicht bestimmte Zustände des Neugeborenen eine höhere Druckbeaufschlagung erfordern. In einer Variante beträgt der intraamniale Druck in dem Raum bzw. Lumen der künstlichen Gebärmutter > 0 mBar und entspricht vorzugsweise dem intraamnialen Druck einer natürlichen Gebärmutter einer lebenden Mutter.

Das Verfahren ist dadurch gekennzeichnet, dass ein Portsystem bereitgestellt wird, das mit einem Oxygenator für die Sauerstoffversorgung und der Dialyseeinrichtung verbunden ist. Dabei besteht das Portsystem aus wenigstens zwei Kathetern zur Verbindung der Umbilikalvene und einer Nabelschnurarterie und mit einem mit den Kathetern verbundenen Stent, der zur Verankerung des Portsystems an die Nabelschnur dient.

### Kurze Beschreibung der Zeichnungen:

Die Erfindung wird in den nachfolgenden Zeichnungen näher erläutert. Es zeigt
- Fig. 1: eine Übersicht einer Ausführungsform des erfindungsgemäßen künstlichen Gebärmuttersystems,
- Fig. 2: das erfindungsgemäße Portsystem,
- Fig. 3: einen Querschnitt einer katheterisierten Nabelschnur eines Frühgeborenen.

### Wege zur Ausführung der Erfindung und gewerbliche Verwertbarkeit:

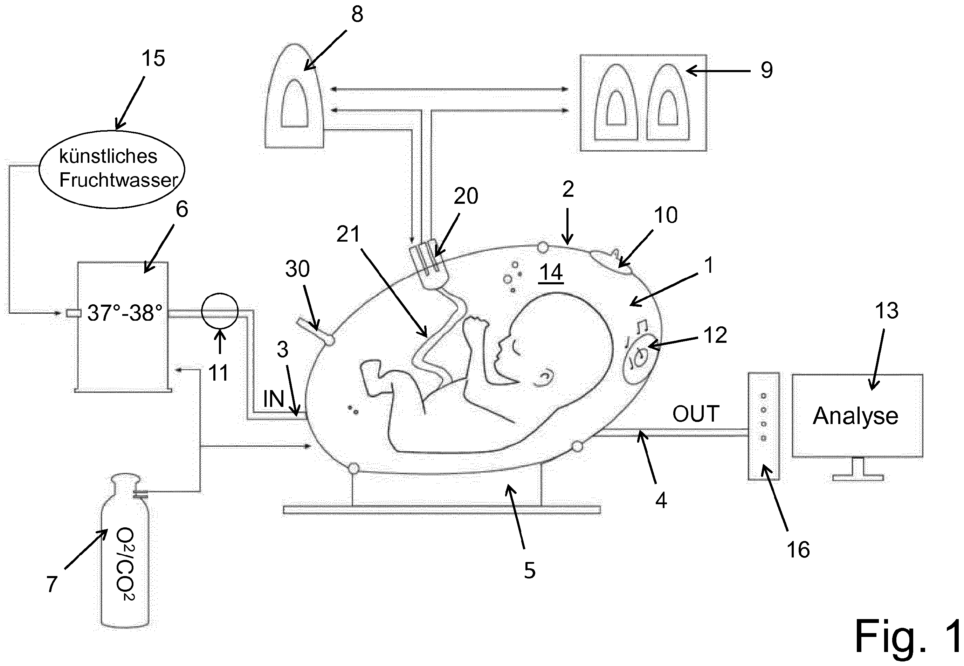

In Fig. 1 sind die erfindungsgemäße Vorrichtung und ihre einzelnen Bestandteile gezeigt.

Das künstliche Gebärmuttersystem umfasst einen von einer zumindest teilweise ultraschalldurchlässigen Wandung 2 gebildeten künstlichen Raum 1 der künstlichen Gebärmutter mit einem Lumen 14 zur Aufnahme von Fruchtwasser 15 und eines Frühgeborenen. Das Fruchtwasser 15 kann beispielsweise in einem Vorratsbehälter bereitgestellt werden, der vorzugsweise über eine Wärmeeinrichtung verfügt, über die das Fruchtwasser 15 vorgewärmt werden kann. Typischerweise erfolgt die Vorwärmung auf eine Temperatur zwischen 38 und 39° C, wobei auch höhere Temperaturen, je nach Ausführung der Leitungen, des Behälters oder anderer Anlagenbestandteile, möglich sind. Vorzugsweise erfolgt die Vorwärmung des Fruchtwassers über eine Fruchtwasservorwärmeinrichtung 6, mit der das dem Raum 1 der künstlichen Gebärmutter zuführende Fruchtwasser 15 auf eine Temperatur von bis zu 39° C vorgewärmt wird. Das Fruchtwasser gelangt über eine Pumpe 11 letztendlich über einen Zugang 3 in den Raum 1 der künstlichen Gebärmutter.

Ein erfindungsgemäßes Portsystem 20 verbindet eine Umbilikalvene sowie eine Nabelschnurarterie einer Nabelschnur 21 mit einem Oxygenator 8. Eine weitere Nabelschnurarterie wird mit einem Oxygenator 9 verbunden. In einer bevorzugten Variante kommunizieren der Oxygenator 8 und die Dialyseeinrichtung 9 miteinander. Der Oxygenator 8 sorgt für eine Sauerstoffversorgung, bedarfsweise mit oder ohne Pumpe. Bevorzugt wird eine ECMO/ECLS-Versorgung durchgeführt. Allein die Versorgung des Frühgeborenen über das erfindungsgemäße Portsystem 20 führte zu einer signifkanten Steigerung der Überlebensrate bei den behandelten Föten. Vorzugsweise kommt bei der ECMO-Anwendung gespendetes Nabelschnurblut zur Anwendung, was auch einer Absenkung des Hb-Gehaltes entgegenwirkt. Auch die Substitution von Ernährungs- und Wachstumsfaktoren kann teilweise über das gespendete Nabelschnurblut verbessert werden.

Vorzugsweise ist die Wandung 2 des Raums 1 der künstlichen Gebärmutter transparent ausgestaltet, sollte jedoch deutlich lichtgedämpft und ultraschalldurchlässig sein. Durch eine ovale Öffnung 10 lässt sich der Raum der künstlichen Gebärmutter 1 von oben öffnen. Ferner ist der Raum 1 der künstlichen Gebärmutter sterilisierbar und das Lumen 14 hat vorzugsweise ein Volumen von 2 bis 5 I. Es werden verschiedene Zugänge 30 bereitgestellt, beispielsweise zum Anschluss von ECMO-Schläuchen, zur Fruchtwasserbegasung oder zur Nährstoffversorgung. Die Wandung 2 des Raums 1 ist in einer bevorzugten Variante doppelseitig ausgebildet, um eine Fruchtwassertemperatur von 37 bis 38° aufrechtzuerhalten.

Die künstliche Gebärmutter mit dem Raum 1 der künstlichen Gebärmutter soll vorzugsweise einen Druck im Falle einer hypobarischen Oxygenierung (HBO) bis zu 2 bar standhalten können. Üblicherweise beträgt der intraamniale Druck zwischen 5 mBar uns 100 mBar. Etwaige Druckmessgeräte können in der Wandung 2 des Raums 1 der künstlichen Gebärmutter integriert sein. Für die künstliche Beatmung ist ab der 23./24. SSW ein Zugang 30 vorgesehen. Vorzugsweise ist in einer Variante des Gebärmuttersystems vorgesehen, dass in der künstlichen Gebärmutter die Sauerstoffversorgung des Neugeborenen über eine Beatmung erfolgt. Das dem Raum 1 der künstlichen Gebärmutter zugeführte Fruchtwasser 15 wird vorzugsweise mit einem Gasgemisch 7 (Carbogen^{®}-Gasgemisch) bestehend aus etwa 5 Volumenteilen Kohlenstoffdioxid und 95 Volumenteilen Sauerstoff, oder O₂ beaufschlagt. Alternativ kann die Beaufschlagung des Carbogen^{®}-Gasgemisches oder O₂ 7 auch direkt in das Fruchtwasser 15 des Gebärmuttersystems erfolgen.

Über eine Messzuleitung 4 kann eine Probennahme für eine Messeinrichtung 16 vorgenommen werden, um beispielsweise die pO₂ in dem Fruchtwasser 15 des Raums 1 der künstlichen Gebärmutter, des Druckes und/oder der Temperatur, O2-Sätigung des Föten 21 zu erfassen. Ferner kann auch eine Sonographie vorgenommen werden. Die Analyse der Messdaten der Messeinrichtung 16 erfolgt über eine Analyseeinrichtung 13. Mütterliche Geräusche können über eine Audioeinrichtung 12 in den Raum 1 der künstlichen Gebärmutter eingespielt werden.

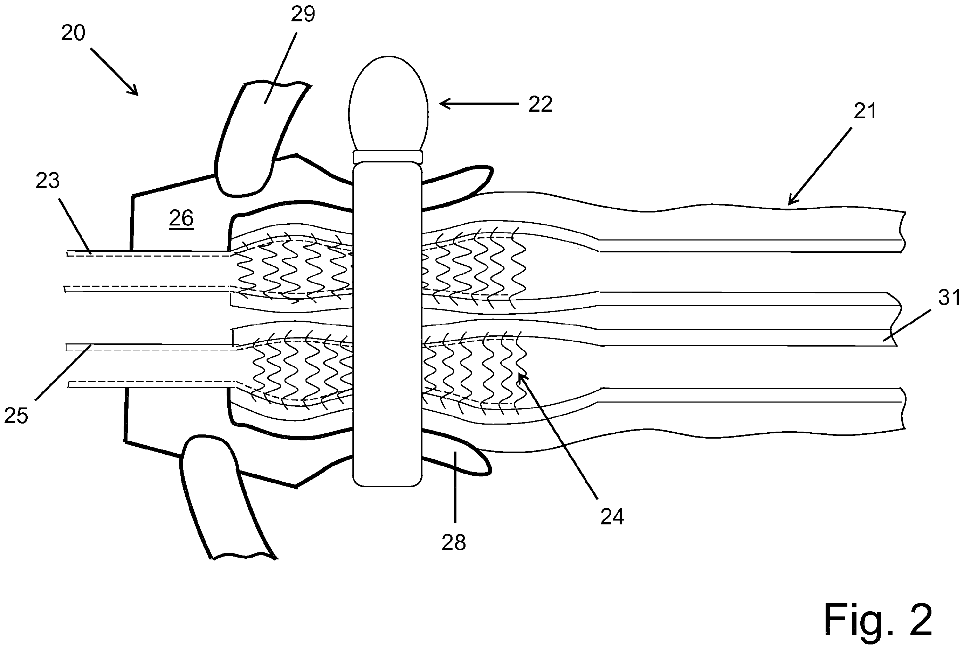

In Fig. 2 ist das erfindungsgemäße Portsystem zur Ansicht im Detail gezeigt. Das Portsystem 20 wird über einen Halter 26 mit der Nabelschnur 21 des Frühgeborenen verbunden. Der Halter 26 umgreift die Außenseite der Nabelschnur 21 voll umfänglich. In die Umbilikalvene wird ein erster Katheter 25 eingeführt. Ein weiterer Katheter 23 wird in eine Nabelschnurarterie eingeführt. Sofern beide Nabelschnurarterien katheterisiert werden sollen, umfasst das erfindungsgemäße Portsystem 20 einen ersten Katheter 25 für die Umbilikalvene und zwei weitere Katheter 23 für die beiden Nabelschnurarterien. Die Umbilikalvene und eine Nabelschnurarterie dienen zur Sauerstoffversorgung und sind mit dem Oxygenator 8 verbunden. Die zweite Nabelschnurarterie ist mit der Dialyseeinrichtung 9 zur Entsorgung des Harns verbunden. Ein Frühgeborenes trinkt ca. 250 bis 300 ml/kg Fruchtwasser, so dass die Entsorgung fetaler Metaboliten (beispielsweise Bilirubin, CO₂) essentiell ist. Die Nabelschnur 21 und der Halter 26 des Portsystems 20 werden über eine Klammer 22 gehalten. Der Halter 26 umfasst vorzugsweise einen fingerartigen Vorsprung 28, der die Nabelschnur 21 und die darin integrierten Blutgefäße aufnimmt und der Presskraft der Klammer 22 standhält. Dargestellt ist auch ein Teil der Gebärmutterwand 29.

Erfindungsgemäß ist vorgesehen, dass die Katheter 23, 25 einen Stent 24 aufweisen, der in der gezeigten Variante aus Greifelementen oder Häkchen besteht, die kraftschlüssig in die Gefäßinnenwand eines Blutgefäßes 31 der Nabelschnur 21 greifen.

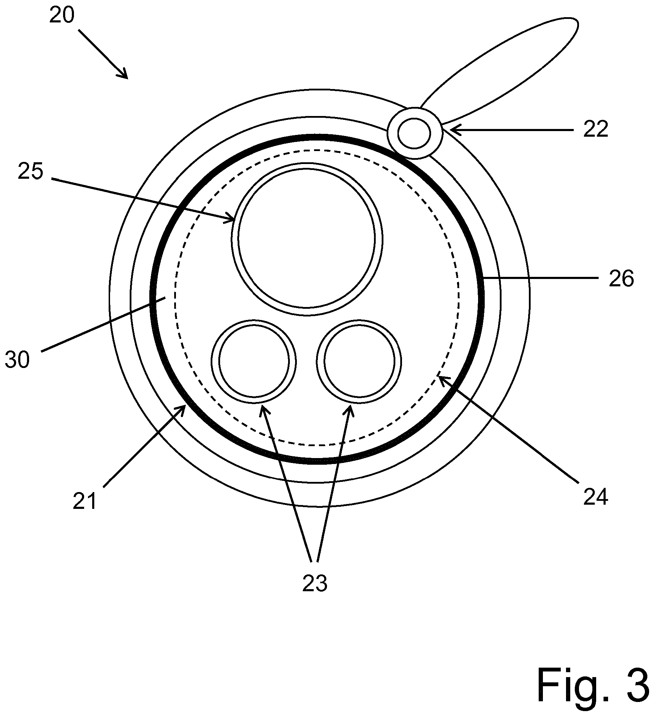

In Fig. 3 erkennt man das erfindungsgemäße Portsystem 20 im Querschnitt. Zu erkennen ist der Katheter 25 für die Umbilikalvene sowie die beiden Katheter 23 für die Nabelschnurarterien. Ferner erkennt man den aus einem Drahtgeflecht bestehenden Stent 24, der um die Katheter 23, 25 umgelegt ist. Der Stent 24 fixiert das Portsystem 20 mit der Gefäßinnenwand des Blutgefäßes 30. Eine zusätzliche Klammer 22 sorgt für die Fixierung des Portsystems 20 über den Halter 26.

### Ausführungsbeispiele:

Das erfindungsgemäße künstliche Gebärmuttersystem wurde in der Klinik im Tiermodel erfolgreich getestet. Dabei haben sich die Vorteile des Portsystems 20 sowie die Aufrechterhaltung des Druckes im Lumen 14 des Gebärmuttersystems zwischen 5 mbar und 100 mBar als besonders vorteilhaft erwiesen, um die physiologische fetale O₂-Sätigung konstant zu halten, Lebenserwartung von extrem Frühgeborenen zu erhöhen und die Morbiditätsrate zu vermindern. Ferner wurde die Anzahl von Sepsis-Fällen deutlich reduziert, da über das erfindungsgemäße Portsystem 20 ein Katheterwechsel der beiden Katheter 23, 25 durch die Kürzung der Nabelschnur ohne weiteres schnell ausführbar ist. Das betreffende Nabelschnurstück der Nabelschnur 21 wird bei der Re-Katheterisierung entfernt, so dass neue Katheter 23, 25 gesetzt werden können. Die Nabelschnur 21 ist ausreichend lang, um mehrere Re-Katheterisierungen durchführen zu können. Durch die sterilen Verhältnisse des künstlichen Gebärmuttersystems wurde auch der Fall einer späten ("late onset") Sepsis" in den Griff bekommen, die bei ca. 40 % von Frühgeborenen in der 25. SSW vorkommt (Stoll et al., Trends in Care Practices, Morbidity, and Mortality of Extremely Preterm Neonates, JAMA, 2015).

Das erfindungsgemäße Gebärmuttersystem ermöglicht ferner einen zügigen Fruchtwasserwechsel bei gleichzeitiger Konstanthaltung der Temperatur und des Druckes in dem Gebärmuttersystem. Über ein Durchspülen ("flush out") mit 2 I bis 20 I Fruchtwasser pro Tag wird eine bakterielle und/oder Pinz-Kolonisierung des Gebärmuttersystems, Nabelschnur, fetaler Haut und Schleimhaut verhindert. Auch die fetalen Abfallprodukte, die über die fetale Haut und Schleimhaut ausgeschieden werden, können mit dem künstlichen Fruchtwasser aus dem Raum abtransportiert werden.

Eine zusätzliche Begasung des Fruchtwassers kann dazu genutzt werden, um die Sauerstoffversorgung des fetalen Gewebes zu erhalten, da die fetale Haut extrem dünn und gasdurchlässig ist. Dabei kann, wenn erforderlich, die CO₂-Konzentration in der Mischung auf einen Wert von kleiner 5 % reduziert werden.

Um die physiologische O₂-Sättigung beim Frühgeborenen dauerhaft garantieren zu können, wird unter sanften hyperbarischen Bedingungen 5 mBbar bis 100 mBar die Begasung im geschlossenen Gebärmuttersystem erfolgen. Eine ausreichende Oxygenierung ist insbesondere während des regelmäßigen Wechsels/ Erneuerns der Apparatur und/oder des Kathetersystems für das frühgeborene Kind wichtig.

Das in die künstliche Gebärmutter zugeführte Fruchtwasser umfasst vorzugsweise eine Zusammensetzung, die dem Entwicklungsstand des Frühgeborenen angepasst ist. Dabei kann Fruchtwasser mit oder ohne Surfaktant/Glucose zur Anwendung kommen. In der eingesetzten Variante umfasst das Fruchtwasser folgende Zusammensetzung:
Na 143.8 mmol/l, K 3,9 mmol/l, Ca 1.6 mmol/l, Mg 0.57 mmol/l, CI-109.5 mmol/l, P 3.3 mg/dl, lactate 9.1 mmol/l, citrate 66.5 mg/dl, HCO 16.9 mmol/l, Cu 16 µg/dl, Se < 13.3 µg/dl, Zn 10-24 µg/dl, pH 8.0, Osmolarität < 271.

Andere Spurenelemente wie z.B. Bor, Chrom, Eisen, Fluor, Jod, Kobalt, Lithium, Mangan, Molybdän, Nickel, Silicium und Vanadium, Aminosäuren, Wachstumsfaktoren, Vitamine und Hormone können das künstliche Fruchtwasser ergänzen.

Das Fruchtwasser wird mit einem Carbogen^{®}-Gas-Gemisch oder O₂ zum Fruchtwassertausch in der künstlichen Gebärmutter oder in einem separaten Gefäß begast. Die Temperatur wurde zwischen 37° und 39° C aufrechterhalten, mit Ausnahme bei der Körperkühlung nach einer Asphyxie. Nach 4 bis 6 Stunden erfolgt ein Austausch des Fruchtwassers. Ein kompletter Austausch sollte spätestens nach 24 Stunden erfolgen, um Infektionen und Kolonisierung zu vermeiden. Das über einen Auslass abgepumpte künstliche Fruchtwasser wird zur Analyse der Nierenfunktion verwendet. Das mit einem Sauerstoff-/ Carbogen^{®} begaste Fruchtwasser kann auch in einem kontinuierlichen Regime im Rahmen einer "flush out"-Anwendung bis 20-30 l/die eingesetzt werden.

Die Möglichkeit zum Abschneiden der Nabelschnur 21 im Rahmen der Fixierung des erfindungsgemäßen Portsystems 20 stellt eine wirksame Methode dar, um eine aseptische Drucknekrose, bakterielle und Pilz-Kolonisierung und Infektionen von Frühgeborenen zu vermeiden. In den klinischen Versuchen wurde eine wöchentliche Reduktion der Nabelschnurlänge um ca. 5 cm durchgeführt, ohne Komplikationen.

Die Kombination der einzelnen physiologischen Parameter, insbesondere die Verbindung eines Oxygenators 8 mit dem erfindungsgemäßen Portsystem 20, die Verbindung mit einer Dialyseeinrichtung 9 und die Aufrechterhaltung eines an die amnialen Bedingungen angelehnten Druckes in dem Raum des künstlichen Gebärmuttersystems führt zu einer deutlich verbesserten Situation und Lebenserhaltung von extrem Frühgeborenen.

## Patentansprüche

1. Künstliches Gebärmuttersystem zur Lebenserhaltung von Neugeborenen, insbesondere von extrem Frühgeborenen zwischen der 21/0 und 28/0 Schwangerschaftswoche, umfassend:
- einen von einer zumindest teilweise ultraschalldurchlässigen Wandung (2) gebildeten Raum (1) der künstlichen Gebärmutter mit einem Lumen (14) zum Erhalten eines physiologischen intraamnialen Druckes sowie zur Aufnahme des künstlichen Fruchtwassers (15) und eines Neugeborenen oder Frühgeborenen,
- wenigstens einen Zugang zur Versorgung des Frühgeborenen in der künstlichen Gebärmutter mit Nährstoffen,
- einen Oxygenator (8) und/oder eine Begasungseinrichtung zur Sauerstoffversorgung des Neugeborenen oder Frühgeborenen,
wobei
- Mittel vorgesehen sind, um einen auf das Neugeborene wirkenden intraamnialen Druck in dem Raum (1) der künstlichen Gebärmutter von >0 mBar zusätzlich zum Atmosphärendruck aufrechtzuerhalten, wobei es sich bei den Mitteln um Begasungsmittel und/oder Fruchtwasser handelt, das auf das Neugeborene in den Raum (1) einwirkt, **dadurch gekennzeichnet dass** eine Dialyseeinrichtung (9) vorgesehen ist und
- der Oxygenator (8) und die Dialyseeinrichtung (9) über ein Portsystem (20) mit der Nabelschnur (21) des Neugeborenen oder Frühgeborenen verbunden sind wobei das Portsystem (20) einen ersten Katheter (25) zur Verbindung der Umbilikalvene und wenigstens einen weiteren Katheter (23) zur Verbindung wenigstens einer Nabelschnurarterie des Neugeborenen oder Frühgeborenen und einen Stent (24) zur Fixierung umfasst.

2. Künstliches Gebärmuttersystem nach Anspruch 1, **dadurch gekennzeichnet, dass** eine Pumpe (11) vorgesehen ist, mit der Fruchtwasser (15) oder Sauerstoff in den Raum (1) der Gebärmutter zur Aufrechterhaltung eines intraamnialen Druckes >0 mBar, vorzugsweise zwischen 2 mBar und 1000 mBar, zusätzlich zum Atmosphärendruck transportiert wird.

3. Künstliches Gebärmuttersystem nach einem der Ansprüche 1 bis 2 **dadurch gekennzeichnet, dass** die Katheter (23, 25) des Portsystems (20) durch einen Halter (26) geführt sind, der so ausgeführt ist, dass er die Nabelschnur (21) des Neugeborenen oder Frühgeborenen von der Außenseite vollumfänglich umgreift und dass ferner der mit den Kathetern (23, 25) verbundene Stent (24) so ausgeführt ist, dass die Katheter (23, 25) mit einer Gefäßinnenwand eines Blutgefäßes (30) der Nabelschnur (21) verbindbar sind.

4. Künstliches Gebärmuttersystem nach einem der Ansprüche 1 bis 3, **dadurch gekennzeichnet, dass** der Stent (24) eine Vielzahl von Häkchen zur Fixierung des Portsystems (20) über die Gefäßinnenwand des Blutgefäßes (30) umfasst.

5. Künstliches Gebärmuttersystem nach einem der Ansprüche 1 bis 4, **dadurch gekennzeichnet, dass** eine Temperaturkontrolleinrichtung vorgesehen ist, mit der eine konstante Temperatur zwischen 36,5 und 37,5 °C in dem Raum (1) der künstlichen Gebärmutter aufrechterhalten wird.

6. Künstliches Gebärmuttersystem nach einem der Ansprüche 1 bis 5, **dadurch gekennzeichnet, dass** das System Anschlüsse für ECMO/ECLS-Schläuche zur Fruchtwassereinleitung und Fruchtwasserableitung, zur Fruchtwasserbegasung und/oder zur Ableitung der Abfallstoffen und Urin des Neugeborenen oder Frühgeborenen umfasst.

7. Künstliches Gebärmuttersystem nach einem der Ansprüche 1 bis 6, **dadurch gekennzeichnet, dass** der Raum (1) der künstlichen Gebärmutter künstliches Fruchtwasser (15) umfasst, welches folgende Zusammensetzung aufweist:
| | Konz. |
|---|---|
| Osmolarität [mosm/l] | 240-300 |
| Natrium [mmol/l] | 130-145 |
| Kalium [mmol/l] | 3,5-4,5 |
| Calcium [mmol/l] | 1-2,5 |
| Magnesium [mmol/l] | 0-2 |
| Chlorid [mmol/l] | 100-120 |
| Harnstoff [mg/dl] | 0-10 |
| Harnsäure [mg/dl] | 0-5 |
| Phosphat [mg/dl] | 0-8 |
| Glucose [mg/dl] | 0-340 |
| Laktat [mmol/l] | 0-20 |
| Citrat [mg/l] | 0-100 |
| Hydrogencarbonat [mmol/l] | 5-100 |
| Gesamt-Eiweiß [g/l] | 0-10 |
| Albumin [g/dl] | 0-5 |
| Kupfer [µg/dl] | 0-150 |
| Selen [µg/dl] | 0-50 |
| Zink [µg/dl] | 0-30 |
| Lipide insgesamt [mg/l] | 0-2000 |
| Phosphatidylcholin (PC) [mg/l] (bevorzugt 80% der Lipide insgesamt) | 0-1000 |
vorzugsweise weiter umfassend Bor, Chrom, Eisen, Fluor, Jod, Kobalt, Lithium, Mangan, Molybdän, Nickel, Silicium und Vanadium, Aminosäuren, Wachstumsfaktoren, Vitamine und/der Hormone.

8. Künstliches Gebärmuttersystem nach einem der Ansprüche 1 bis 7, **dadurch gekennzeichnet, dass** der Oxygenator (8) den Raum (1) der künstlichen Gebärmutter oder das dem Raum (1) zugeführte Fruchtwasser (15) mit einem Gasgemisch (7), bestehend aus etwa 5 Volumenteilen Kohlenstoffdioxid (CO₂) und 95 Volumenteilen Sauerstoff (O₂) oder nur O₂, beaufschlagt.

9. Künstliches Gebärmuttersystem nach einem der Ansprüche 1 bis 8, **dadurch gekennzeichnet, dass** ferner eine Messeinrichtung (16) zur Erfassung der O₂ Sättigung der Frühgeborenen, pO₂ in dem Fruchtwasser (15) des Systems, des Druckes und/oder der Temperatur in der künstlichen Gebärmutter oder zur Sonografie vorgesehen ist.

10. Künstliches Gebärmuttersystem nach einem der Ansprüche 1 bis 9, **dadurch gekennzeichnet, dass** eine Fruchtwasservorwärmeinrichtung (6) vorgesehen ist, mit der das dem Raum (1) zuzuführende Fruchtwasser (15) auf eine Temperatur von bis zu 39 °C vorwärmbar ist.

11. Künstliches Gebärmuttersystem nach einem der Ansprüche 1 bis 10, **dadurch gekennzeichnet, dass** die künstliche Gebärmutter auf einer Bewegungseinrichtung und/oder Waage gelagert ist.

## Claims

1. An artificial womb system for supporting newborns, in particular extremely premature infants between the 21/0 and 28/0 week of gestation, comprising:
- a chamber (1) of the artificial womb, said chamber (1) being formed by an at least partially ultrasound-permeable wall (2) and comprising a lumen (14) for maintaining a physiologically intraamnial pressure and for receiving the artificial amniotic fluid (15) and a newborn or a premature infant,
- at least one access for supplying the premature infant in the artificial womb with nutrients,
- an oxygenator (8) and/or a gassing device for supplying oxygen to the newborn or premature infant,
whereby means are provided in order to maintain an intraamnial pressure of > 0 mbar in addition to the atmospheric pressure acting on the newborn in the chamber (1) of the artificial womb, the means being fumigants and/or amniotic fluid acting on the newborn into the room (1), **characterized in that** a dialysis device (9) is provided and
- the oxygenator (8) and the dialysis device (9) are connected to the umbilical cord (21) of the newborn or premature infant via a port system (20), the port system (20) comprising a first catheter (25) for connecting the umbilical vein and at least one further catheter (23) for connecting at least one umbilical artery of the newborn or premature infant and a stent (24) for fixation.

2. The artificial womb system according to claim 1, **characterized in that** a pump (11) is provided, which feeds amniotic fluid (15) or oxygen into the chamber (1) of the womb to maintain an intraamnial pressure of > 0 mbar, preferably between 2 mbar and 1000 mbar, in addition to the atmospheric pressure.

3. The artificial womb system according to any one of claims 1 to 2, **characterized in that** the catheters (23, 25) of the port system (20) are guided by a holder (26) designed to fully encompass the umbilical cord (21) of the newborn or preterm infant from the outside and **characterized in that** the stent (24) connected to the catheters (23, 25) is designed in such a way that the catheters (23, 25) can be connected to the endangium of a blood vessel (30) of the umbilical cord (21).

4. The artificial womb system according to any one of claims 1 to 3, **characterized in that** the stent (24) comprises a number of fasteners for fixing the port system (20) via the endangium of the blood vessel (30).

5. The artificial womb system according to any one of claims 1 to 4, **characterized in that** a temperature control device is provided in order to maintain a constant temperature between 36.5 °C and 37.5 °C in the chamber (1) of the artificial womb.

6. The artificial womb system according to any one of claims 1 to 5, **characterized in that** the system provides accesses for ECMO/ECLS tubes, for amniotic fluid inlet and amniotic fluid outlet, for amniotic fluid gassing and/or for the discharge of metabolic waste and urine of the newborn or premature infant.

7. The artificial womb system according to any one of claims 1 to 6, **characterized in that** the chamber (1) of the artificial womb comprises artificial amniotic fluid (15), which has the following composition:
| | Concentration |
|---|---|
| Osmolarity [mosm/l] | 240-300 |
| Sodium [mmol/l] | 130-145 |
| Potassium [mmol/l] | 3,5-4,5 |
| Calcium [mmol/l] | 1-2,5 |
| Magnesium [mmol/l] | 0-2 |
| Chloride [mmol/l] | 100-120 |
| Urea [mg/dl] | 0-10 |
| Uric acid [mg/dl] | 0-5 |
| Phosphate [mg/dl] | 0-8 |
| Glucose [mg/dl] | 0-340 |
| Lactate [mmol/l] | 0-20 |
| Citrate [mg/l] | 0-100 |
| Hydrogencarbonate [mmol/l] | 5-100 |
| Total protein [g/l] | 0-10 |
| Albumin [g/dl] | 0-5 |
| Copper [µg/dl] | 0-150 |
| Selenium [µg/dl] | 0-50 |
| Zinc [µg/dl] | 0-30 |
| Total lipids [mg/l] | 0-2000 |
| Phosphatidylcholin (PC) [mg/l] (preferably 80 % of total lipids) | 0-1000 |
preferably further comprising boron, chromium, iron, fluorine, iodine, cobalt, lithium, manganese, molybdenum, nickel, silicon and vanadium, amino acids, growth factors, vitamins and/or hormones.

8. The artificial womb system according to any one of claims 1 to 7, **characterized in that** the oxygenator (8) infuses the chamber (1) of the artificial womb or the amniotic fluid (15) led into the chamber (1) with a gas mixture (7) consisting of about 5 volumes of carbon dioxide (CO₂) and 95 volumes of oxygen (O_{2,}) or with O₂ only.

9. The artificial womb system according to any one of claims 1 to 8, **characterized in that** it further comprises a measuring device (16) for monitoring the O₂-saturation in premature infants, pO₂ in the amniotic fluid (15) of the system, the pressure and/or the temperature in the artificial womb or intended for sonography.

10. The artificial womb system according to any one of claims 1 to 9, **characterized in that** an amniotic fluid pre-heating device (6) is provided, which may pre-heat the amniotic fluid (15) to a temperature of up to 39 °C before entering the chamber (1).

11. The artificial womb system according to any one of claims 1 to 10, **characterized in that** the artificial womb is mounted on a moving device and/or scale.

## Revendications

1. Système d'utérus artificiel destiné au maintien en vie de nouveau-nés, en particulier de prématurés extrêmes entre 21/0 et 28/0 semaines de grossesse, ledit système comprenant :
- un espace (1) de l'utérus artificiel qui est formé par une paroi (2) au moins partiellement transparente aux ultrasons et qui comporte une lumière (14) destinée à maintenir une pression intra-amniale physiologique et à recevoir le liquide amniotique artificiel (15) et un nouveau-né ou prématuré,
- au moins un accès destiné à alimenter en nutriments le prématuré se trouvant dans l'utérus artificiel,
- un oxygénateur (8) et/ou un dispositif d'apport de gaz destiné à fournir de l'oxygène au nouveau-né ou au prématuré,
- des moyens étant prévus pour maintenir une pression intra-amniale, agissant sur le nouveau-né, de > 0 mbar dans l'espace (1) de l'utérus artificiel en plus de la pression atmosphérique, les moyens étant des moyens d'apport de gaz et/ou du liquide amniotique qui agissent sur le nouveau-né dans l'espace (1), **caractérisé en ce qu'**un dispositif de dialyse (9) est prévu et
- l'oxygénateur (8) et le dispositif de dialyse (9) sont reliés au cordon ombilical (21) du nouveau-né ou du prématuré par le biais d'un système d'orifices (20), le système d'orifices (20) comprenant un premier cathéter (25) destiné à être relié à la veine ombilicale et au moins un autre cathéter (23) destiné à être relié à au moins une artère ombilicale du nouveau-né ou du prématuré et un stent (24) destiné à la fixation.

2. Système d'utérus artificiel selon la revendication 1, **caractérisé en ce qu'**une pompe (11) est prévue qui transporte le liquide amniotique (15) ou l'oxygène jusque dans l'espace (1) de l'utérus pour maintenir une pression intra-amniale > 0 mbar, de préférence entre 2 mbar et 1000 mbar, en plus de la pression atmosphérique.

3. Système d'utérus artificiel selon l'une des revendications 1 et 2, **caractérisé en ce que** les cathéters (23, 25) du système d'orifices (20) sont guidés à travers un support (26) qui est conçu de manière à s'engager entièrement autour du cordon ombilical (21) du nouveau-né ou du prématuré depuis le côté extérieur et **en ce que** le stent (24), relié aux cathéters (23, 25), est conçu de manière à ce que les cathéters (23, 25) puissent être reliés à une paroi interne d'un vaisseau sanguin (30) du cordon ombilical (21).

4. Système d'utérus artificiel selon l'une des revendications 1 à 3, **caractérisé en ce que** le stent (24) comprend un grand nombre de crochets destinés à fixer le système d'orifices (20) sur la paroi interne du vaisseau sanguin (30).

5. Système d'utérus artificiel selon l'une des revendications 1 à 4, **caractérisé en ce qu'**un dispositif de commande de température est prévu, qui maintient une température constante entre 36,5 et 37,5 °C dans l'espace (1) de l'utérus artificiel.

6. Système d'utérus artificiel selon l'une des revendications 1 à 5, **caractérisé en ce que** le système comprend des raccords destinés à des tubes ECMO/ECLS pour l'introduction et le drainage du liquide amniotique, pour l'apport de gaz dans le liquide amniotique et/ou pour le drainage des déchets et de l'urine du nouveau-né ou du prématuré.

7. Système d'utérus artificiel selon l'une des revendications 1 à 6, **caractérisé en ce que** l'espace (1) de l'utérus artificiel comprend du liquide amniotique artificiel (15) qui a la composition suivante :
| | conc. |
|---|---|
| Osmolarité [mosm/l] | 240 à 300 |
| Azote [mmol/l] | 130 à 145 |
| Potassium [mmol/l] | 3,5 à 4,5 |
| Calcium [mmol/l] | 1 à 2,5 |
| Magnésium [mmol/l] | 0 à 2 |
| Chlorure [mmol/l] | 100 à 120 |
| Urée [mg/dl] | 0 à 10 |
| Acide urique [mg/dl] | 0 à 5 |
| Phosphate [mg/dl] | 0 à 8 |
| Glucose [mg/dl] | 0 à 340 |
| Lactate [mmol/l] | 0 à 20 |
| Citrate [mg/l] | 0 à 100 |
| Hydrogénocarbonate [mmol/1] | 5 à 100 |
| Albumine total [g/l] | 0 à 10 |
| Albumen [g/dl] | 0 à 5 |
| Cuivre [µg/dl] | 0 à 150 |
| Sélénium [µg/dl] | 0 à 50 |
| Zinc [µg/dl] | 0 à 30 |
| Lipides totaux [mg/1] | 0 à 2000 |
| Phosphatidylcholine (PC) [mg/l] (de préférence 80 % des lipides totaux) | 0 à 1000 |
comprenant en outre de préférence du bore, du chrome, du fer, du fluor, de l'iode, du cobalt, du lithium, du manganèse, du molybdène, du nickel, du silicium et du vanadium, des acides aminés, des facteurs de croissance, des vitamines et/ou des hormones.

8. Système d'utérus artificiel selon l'une des revendications 1 à 7, **caractérisé en ce que** l'oxygénateur (8) soumet l'espace (1) de l'utérus artificiel ou le liquide amniotique (15) amené à l'espace (1) à un mélange gazeux (7) comprenant environ 5 parties en volume de dioxyde de carbone (CO₂) et 95 parties en volume d'oxygène (O₂) ou seulement Oz.

9. Système d'utérus artificiel selon l'une des revendications 1 à 8, **caractérisé en ce qu'**un dispositif de mesure (16) est également prévu pour détecter la saturation en O₂ des prématurés, la pO₂ dans le liquide amniotique (15) du système, la pression et/ou la température dans l'utérus artificiel ou pour l'échographie.

10. Système d'utérus artificiel selon l'une des revendications 1 à 9, **caractérisé en ce qu'**un dispositif de préchauffage de liquide amniotique (6) est prévu, qui préchauffe le liquide amniotique (15) à amener à l'espace (1) à une température pouvant atteindre 39 °C.

11. Système d'utérus artificiel selon l'une des revendications 1 à 10, **caractérisé en ce que** l'utérus artificiel est monté sur un dispositif de déplacement et/ou une balance.
